# FASCICULE DE BREVET EUROPEEN

(11) **EP 0 796 615 B1**
(45) Date de publication et mention de la délivrance du brevet: **24.01.2001**
(21) Numéro de dépôt: 97400284.2
(22) Date de dépôt: 07.02.1997
(51) Int. Cl.: A61K 7/50, A61K 7/48, A61K 7/06

(54) **Composition cosmétique détergente contenant une silicone oxyalkylénée**
Reinigende kosmetische Zusammensetzung enthaltend ein oxyalkyliertes Silikon
Cleaning cosmetic composition containing an oxyalkylated silicon

(30) Priorité: 21.03.1996 FR 9603543
(43) Date de publication de la demande: 24.09.1997
(73) Titulaire: L'OREAL, 75008 Paris (FR)
(72) Inventeur: Dubief, Claude, 78150 Le Chesnay (FR); Cauwet-Martin, Danièle, 75011 Paris (FR)
(74) Mandataire: Andral, Christophe André Louis

(56) Documents cités:
- EP-A- 0 155 806
- EP-A- 0 521 748
- EP-A- 0 613 942
- EP-A- 0 633 018
- FR-A- 2 694 494
- US-A- 5 217 652
- SEIFEN-ÖLE-FETTE-WACHSE, vol. 112, no. 4, Mars 1986, AUGSBURG, pages 123-126, XP002023742 ROIDL: "Anwendung von Siliconpolymeren und Siliconen mit funktionellen Gruppen in der Kosmetik"
- FALBE ET AL.: 'Römpp Chemie Lexikon, Seite 4351, 9. Aufl.', 1992,, GEORG THIEME VERLAG, STUTTGART
- FIEDLER HP: 'Lexikon der Hilfsstoffe für Pharmazie, Kosmetik und angrenzende Gebiete', Seite 382, 3. Aufl., 1989, EDITIO CANTER, AULENDORF

## Description

La présente invention concerne de nouvelles compositions cosmétiques destinées au nettoyage des cheveux, du cuir chevelu et/ou de la peau contenant au moins un tensioactif détergent, au moins un cotensioactif, au moins un électrolyte et au moins une silicone oxyalkylénée ainsi que leur utilisation dans cette application cosmétique.

Pour le nettoyage des cheveux et/ou de la peau, l'utilisation de compositions cosmétiques détergentes (shampooings ou gels-douche par exemple) contenant à la fois des agents tensioactifs à pouvoir lavant et un ou plusieurs agents de conditionnement est courante.

En effet, pour améliorer les propriétés cosmétiques des compositions détergentes, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est souvent nécessaire d'introduire dans ces dernières des agents de conditionnement cosmétiques complémentaires comme par exemple des silicones, qui apportent alors aux cheveux traités une facilité de démêlage et de coiffage, ainsi qu'une douceur et une brillance nettement accrues.

De même, il peut être intéressant de traiter le cuir chevelu avec des compositions contenant des actifs tels que des agents anti-pelliculaires.

Compte tenu du caractère insoluble de la plus part des agents de conditionnement ou de certains agents anti-pelliculaires dans les milieux aqueux utilisés dans les shampooings, on cherche à les maintenir sous forme dispersée. Cette forme dispersée permet aux agents de conditionnement ou anti-pelliculaires de se déposer sur les cheveux ou sur la peau et de ne pas être totalement éliminés lors du rinçage. Il importe toutefois que la mise en suspension ne perturbe pas les propriétés détergentes et moussantes de la composition cosmétique.

Il existe à ce jour peu de moyens pour maintenir efficacement en suspension les agents insolubles, car il s'agit là d'un problème difficile à résoudre ; à cet effet, on a déjà proposé l'utilisation des dérivés d'esters ou d'éthers à longue chaîne ou des polysaccharides tels que la gomme de xanthane. Cependant, les esters ou éthers à longue chaîne présentent des problèmes de cristallisation qui entraînent une évolution (augmentation) de la viscosité des compositions au cours du temps ; les polysaccharides gélifiants présentent également des inconvénients, à savoir, d'une part que la mousse des compositions détergentes les contenant se développe difficilement (mauvais démarrage de mousse) et que, d'autre part, les compositions n'ont pas une texture lisse et s'écoulent par paquets, ce qui n'est pas apprécié des utilisateurs.

On a également proposé dans la demande de brevet français 2694494 des compositions contenant en suspension des particules non hydrosolubles contenant un tensioactif anionique, un cotensioactif non ionique ou amphotère et un électrolyte, lesdits tensioactifs étant en une quantité telle que la composition possède un comportement pseudoplastique avec un seuil d'écoulement supérieur à 0,2 Pa et présente une structure de phase lamellaire incluant des sphérulites aptes à maintenir en suspension des particules non hydrosolubles.
Cependant, ces compositions, qui permettent la mise en suspension de particules, présentent des propriétés d'usage non satisfaisantes notamment lorsqu'elles sont utilisées comme shampooing ou gel-douche. En particulier, les propriétés moussantes, telles que le démarrage de la mousse, ne donnent pas entière satisfaction. Les mousses sont généralement trop compactes et difficiles à travailler.
La demanderesse a donc cherché à améliorer les propriétés moussantes de ces compositions.

La présente invention a donc pour but de proposer des compositions cosmétiques détergentes possédant de bonnes propriétés moussantes, aptes à maintenir en suspension des actifs non hydrosolubles.

Ainsi, la demanderesse a maintenant trouvé que ces objectifs étaient atteints en introduisant une silicone oxyalkylénée dans un milieu comprenant au moins un tensioactif anionique, au moins un cotensioactif non ionique ou amphotère et au moins un électrolyte présent à une concentration comprise entre 6 et 15% en poids par rapport au poids total de la composition, et choisi parmi :
i) les nitrates de lithium, de strontium, de barium, d'yttrium, de néodyme, de gadolinium, de manganèse, de zinc ou de magnésium,
ii) les chlorures de lithium, de strontium, de barium, d'yttrium, de néodyme, de gadolinium, de manganèse, de zinc, de magnésium ou de sodium,
iii) les sulfates de calcium, de strontium ou de magnésium,
iv) les acétates de strontium, de manganèse ou de magnésium,
lesdits composés étant en quantité telle que la composition présente :
(a) un comportement rhéologique en écoulement caractérisé par un domaine de contraintes pour lesquelles la viscosité est constante, suivi d'un domaine de contraintes pour lequel la viscosité diminue lorsque la contrainte augmente, et
(b) une structure de phase lamellaire, cette phase étant apte à maintenir en suspension des particules non hydrosolubles éventuellement présentes dans la composition.

Les propriétés moussantes des compositions, telles que le démarrage, l'abondance et la tenue de la mousse, sont nettement améliorées.

Les compositions selon l'invention sont stables : elles permettent de maintenir en suspension des particules liquides (gouttelettes) ou solides non hydrosolubles.

L'invention a donc pour objet une composition cosmétique détergente, caractérisée par le fait qu'elle comprend au moins un tensioactif anionique, au moins un cotensioactif non ionique ou amphotère, au moins une silicone oxyalkylénée et au moins un électrolyte, lesdits composés étant en quantité telle que la composition présente :
(a) un comportement rhéologique en écoulement caractérisé par un domaine de contraintes pour lesquelles la viscosité est constante, suivi d'un domaine de contraintes pour lequel la viscosité diminue lorsque la contrainte augmente, et
(b) une structure de phase lamellaire, cette phase étant apte à maintenir en suspension des particules non hydrosolubles éventuellement présentes dans la composition.

De plus, la composition présente de bonnes propriétés lavantes et des propriétés cosmétiques avantageuses (douceur, démêlage, coiffage).

Le comportement rhéologique en écoulement des compositions est caractérisé à l'aide d'un rhéomètre à contrainte imposée (Carrimed CSHR100). Les mesures sont réalisées à 25°C en utilisant un corps de mesure plan-cone de 2 degrés d'angle et de 6 cm de diamètre.

Les contraintes, pour lesquelles la viscosité d'une composition donnée est constante, sont variables. Généralement, selon la présente invention, elles sont comprises entre 0,001 et 10 Pa et de préférence entre 0,01 et 2 Pa.

Les compositions selon l'invention présentent une phase lamellaire, c'est à dire une phase solide hydratée ou de cristaux liquides dans laquelle plusieurs bicouches sont disposées en réseau parallèle, séparées par des couches d'eau ou de solution aqueuse.
La phase lamellaire peut éventuellement contenir des sphérulites qui sont des vésicules plurilamellaires constituées de plusieurs couches de tensioactifs disposées concentriquement et de dimensions généralement comprises entre 0,1 et 50 micromètres.

L'invention a également pour objet un procédé pour le nettoyage des cheveux, de la peau et/ou du cuir chevelu à l'aide des compositions ci-dessus.

Ainsi, à titre d'exemple de tensioactifs anioniques utilisables, seuls ou en mélange, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, les alkylamidoéthersulfates, les monoglycérides sulfates, les alkylglycérylsulfonates, les alkylsulfonates, les alkylphosphates, les alkylamidesulfonates, les alkylarylsulfonates, les α-oléfinesulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acylaminoacides tels que les N-acylsarcosinates, les N-acylglutamates et les N-acyltaurates. Parmi les tensioactifs anioniques encore utilisables, on peut également citer les sels d'acides gras tels que les sels des acides undécylénique, oléique, ricinoléique, palmitique, stéarique, les acides d'huile de coprah ou d'huile de coprah hydrogénée ; les acylhydroxyacides tels que les acyl-lactylates. On peut également utiliser des tensioactifs faiblement anioniques, comme les acides alkyl-D-galactoside uroniques et leurs sels ainsi que les acides éthers carboxyliques polyoxyalkylénés, en particulier ceux comportant de 2 à 24 groupements oxyde d'éthylène, et leurs mélanges. Le radical alkyle ou acyle de tous ces différents composés comportent de préférence de 8 à 22 atomes de carbones.

A titre de cotensioactifs non ioniques utilisables selon l'invention, on peut citer les alcools, les alphadiols, les alkylphénols ou les acides gras éthoxylés, propoxylés ou glycérolés, ces composés ayant une chaîne grasse comportant de 8 à 28 atomes de carbone, le nombre de groupements d'oxyde d'éthylène ou de propylène pouvant aller de 1 à 50 et celui de glycérol notamment de 1 à 30. On peut également citer les copolymères d'oxyde d'éthylène et de propylène, les condensats d'oxyde d'éthylène et de propylène sur des alcools gras; les amines ou les amides gras polyéthoxylés ayant de préférence de 2 à 30 moles d'oxyde d'éthylène, les amides gras glycérolés comportant en moyenne 1 à 5 groupements glycérol, les diglycolamides polyglycérolés, les esters d'acides gras du sorbitan éventuellement oxyéthylénés, les esters d'acides gras du sucrose, les esters d'acides gras polyoxyalkylénés, les alkylpolyglycosides éventuellement oxyalkylénés, les esters d'alkylglucosides, les dérivés de N-alkylglucamine et de N-acylméthylglucamines, les oxydes d'amine et leurs mélanges.

A titre de cotensioactifs amphotères utilisables selon l'invention, on peut citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer les alkylbétaines, les alkyldiméthylbétaïnes, les alkylsulfobétaïnes, les alkyl amido alkyl bétaïnes, les alkyl amido alkyl sulfobétaïnes, les dérivés d'imidazoline tels que les dérivés d'amphocarboxyglycinate ou d'ampho-carboxypropionate et leurs mélanges.

Les cotensioactifs préférés selon l'invention sont choisis parmi les alkylbétaines et les alkyl amido alkyl bétaïnes.

Le ou les tensioactifs anioniques sont généralement présents dans les compositions conformes à l'invention dans des proportions comprises entre 3 à 50 % en poids, de préférence de 5 à 30 % en poids, par rapport au poids total de la composition.

Le ou les cotensioactifs sont généralement présents dans les compositions conformes à l'invention dans des proportions comprises entre 0,05 et 30 % en poids, de préférence de 1 à 15 % en poids, par rapport au poids total de la composition.

La somme des concentrations en tensioactifs anioniques et cotensioactifs est généralement comprise entre 3 et 70 % en poids par rapport au poids total de la composition.

Le rapport pondéral cotensioactif / tensioactif anionique est de préférence inférieur ou égal à 1 et plus particulièrement compris entre 0,01 et 1 et préférentiellement compris entre 0,05 et 0,75.

On préfère utiliser les électrolytes dont la solubilité dans l'eau est comprise entre. 0,1 g% et 300 g%

Parmi les électrolytes, on peut citer les sels métalliques, les sels d'amines, d'ammonium ou d'aminoacides basiques.

Les sels métalliques sont plus particulièrement choisis parmi les sels de métaux alcalins, de métaux alcalino-terreux, de métaux de transition et de métaux des groupes 13 et 14 de la classification périodique des éléments.

Comme sels de métaux alcalins utiles selon l'invention, on peut citer en particulier les sels de lithium, de sodium ou de potassium.

Comme sels de métaux alcalino-terreux utiles selon l'invention, on peut citer en particulier les sels de béryllium, de magnésium, de calcium, de strontium et/ou de baryum.

Comme sels de métaux de transition utiles selon l'invention, on peut citer en particulier les sels de lanthanides, et les sels de métaux de la quatrième période de la classification périodique des éléments, comme les sels de manganèse, de cobalt ou de zinc.

Comme sels de métaux des groupes 13 et 14 de la classification périodique des éléments utiles selon l'invention, on peut citer les sels d'aluminium et d'étain.

Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium.

De manière préférentielle, les sels métalliques selon l'invention sont choisis parmi les sels de lithium, de strontium, de barium, d'yttrium, de néodyme, de gadolinium, de manganèse et de zinc, plus préférentiellement de strontium.

Ces sels peuvent être par exemple des carbonates, des bicarbonates, des sulfates, des glycérophosphates, des borates, des chlorures, des nitrates, des acétates, des hydroxydes, des persulfates ainsi que des sels d'a-hydroxyacides ou des sels d'acides de fruits (citrate, tartrate, lactate, malate), ou encore des sels d'acides aminés (aspartate, arginate, glucocholate, fumarate) ou des sels d'acides gras (palmitate, oléate, caséinate, béhénate).

De préférence le sel est choisi parmi les nitrates ou les chlorures, en particulier le nitrate de lithium, de strontium, de barium, d'yttrium, de néodyme, de gadolinium, de manganèse ou de zinc, le chlorure de lithium, de strontium, de barium, d'yttrium, de néodyme, de gadolinium, de manganèse ou de zinc, les sulfates ou les acétates, comme le sulfate de calcium, de strontium ou de magnésium et l'acétate de strontium ou de magnésium.

De façon encore plus avantageuse, l'électrolyte est un sel de magnésium ou de strontium, se présentant notamment sous forme de chlorure ou de nitrate.

Selon l'invention, la concentration en électrolyte est de préférence comprise entre 6 et 13% en poids par rapport au poids total de la composition.

Les silicones oxyalkylénées utilisables selon l'invention peuvent être hydrosolubles, spontanément hydrodispersibles ou non hydrosolubles. Elles sont selon l'invention de préférence hydrosolubles ou spontanément hydrodispersibles.

Les silicones oxyalkylénées sont par exemple choisies parmi les composés de formules générales (I), (II), (III), (IV) et (V): 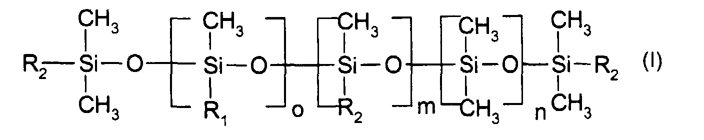 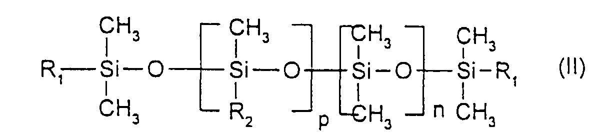 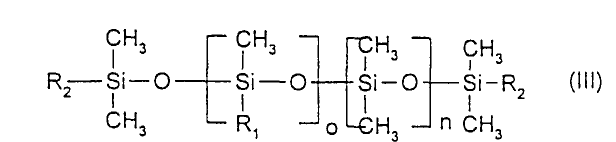 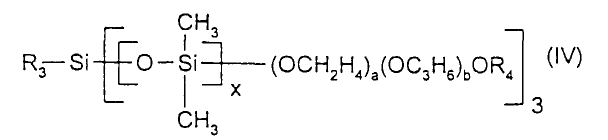 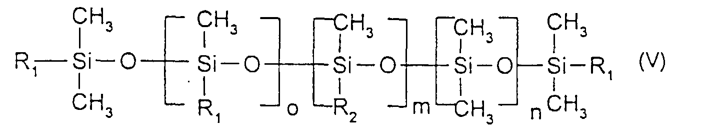 formules dans lesquelles :
- R₁, identique ou différent, représente un radical alkyle, linéaire ou ramifié, en C₁-C₃₀ ou phényle.
- R₂, identique ou différent, représente -C_{c}H_{2c}-O-(-C₂H₄O)ₐ(-C₃H₆O)_{b}-R₅ ou -C_{c}H_{2c}-O-(-C₄H₈O)ₐ-R₅,
- R₃, R₄, identiques ou différents, désignent un radical alkyle, linéaire ou ramifié, en C₁-C₁₂, et de préférence le radical méthyle,
- R₅, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 12 atomes de carbone, un radical hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, N(CH₂CH₂COOM)₂,aminoalkyle éventuellement substitué sur l'amine et sur la chaîne alkyle, carboxyacyle en C₁-C₃₀, un groupement phosphono éventuellement substitué par un ou deux radicaux aminoalkyle substitués, -CO(CH₂)_{d}COOM, -OCOCHR₇(CH₂)_{d}COOM, -NHCO(CH₂)_{d}OH, -NH₃Y,
- M, identique ou différent, désigne un atome d'hydrogène, Na, K, Li, NH₄ ou une amine organique,
- R₆ désigne un radical alkyle, linéaire ou ramifié, en C₁-C₃₀,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 1,
- c varie de 0 à 4,
- x varie de 1 à 100,
- Y représente un anion minéral ou organique monovalent tel que halogénure (chlorure, bromure), sulfate, carboxylate (acétate, lactate, citrate).

De préférence, on utilise les silicones oxyalkylénées répondant aux formules générales (I) ou (II). Plus particulièrement, ces formules répondent à au moins une des, et de préférence toutes les, conditions suivantes :
- c est égal à 2 ou 3.
- R₁ désigne le radical méthyle.
- R₅ représente un atome d'hydrogène, un radical méthyle ou un radical acétyle et de préférence un atome d'hydrogène.
- a varie de 1 à 25 et plus particulièrement de 2 à 15.
- b est égal à 0.
- n varie de 0 à 100.
- p varie de 1 à 20

Les silicones les plus particulièrement préférées sont par exemple celles vendues sous les dénominations commerciales FLUID DC 193 par la société DOW CORNING, SILWET L 77 par la société OSI et MAZIL 756 par la société MAZER PPG.

Les silicones oxyalkylénées sont généralement présentes dans les compositions selon l'invention dans des concentrations comprises entre 0,01 et 10% en poids, et de préférence entre 0,2 et 5 % en poids, par rapport au poids total de la composition.

Les compositions selon l'invention présentent généralement une viscosité supérieure à 200 mPa.s.

Par particules non hydrosolubles, on désigne des entités, solides ou non, qui ne se solubilisent pas dans le milieux aqueux de la composition.

Les particules non hydrosolubles pouvant être éventuellement dispersées dans les compositions selon l'invention sont par exemple les gommes, les résines ou les huiles de silicones modifiées ou non, les composés fluorés, les agents antipelliculaires, les huiles végétales, minérales ou synthétiques, les cires, les agents nacrants, les pigments, les esters d'acide gras, les particules abrasives telles que la silice, les parfums, les polymères insolubles dans l'eau.

Bien entendu, l'homme du métier veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions détergentes selon l'invention présentent un pH final généralement compris entre 3 et 8. De préférence, ce pH est compris entre 4 et 7,5. L'ajustement du pH à la valeur désirée peut se faire classiquement par ajout, selon les cas, soit d'agents basifiants, soit d'agents acidifiants, usuels et connus comme cosmétiquement acceptables.

Les compositions détergentes selon l'invention peuvent bien entendu contenir en outre tous les adjuvants habituellement rencontrés dans le domaine des compositions détergentes pour les cheveux et/ou pour le corps, comme par exemple des parfums, des agents conservateurs, des séquestrants, des agents acidifiants, des agents alcalinisants, des adoucissants, des modificateurs de mousse, des colorants, des agents nacrants, des agents hydratants, des agents antipelliculaires ou anti-séborrhéiques, des vitamines, des silicones, des céramides, des filtres solaires, des polymères cationiques, anioniques, non ioniques ou amphotères.

Ces compositions peuvent se présenter sous la forme de liquides épaissis, de crèmes ou de gels et elles conviennent principalement au lavage des cheveux et/ou de la peau.

L'invention a encore pour objet un procédé de lavage de la peau ou des fibres kératiniques, telles que les cheveux, consistant à appliquer sur ceux-ci une composition telle que définie précédemment, puis à effectuer un rinçage à l'eau.

Des exemples concrets illustrant l'invention vont maintenant être donnés.

Dans ce qui suit MA signifie matière active.

### EXEMPLE 1

On a préparé un shampooing conforme à l'invention qui présente la composition suivante :
- Lauryl éther sulfate de sodium à 2,2 moles d'oxyde d'éthylène en solution aqueuse à 28 % de MA 16,8 g MA
- Cocoyl bétaïne en solution aqueuse à 30 % de MA 2,7 g MA
- Silicone oxyéthylénée de formule (II) ( DC 193 vendu par DOW CORNING) 1 g
- NaCl 12 g
- Conservateurs, colorants, parfum qs
- Eau qsp 100 g

Le pH spontané de la composition est de 5,8.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.

Le shampooing a été appliqué sur les cheveux mouillés, on a obtenu rapidement une mousse onctueuse et agréable.

Un panel de 10 testeurs expérimentés a comparé le pouvoir moussant de cette composition à celui d'une même composition ne contenant pas le diméthicone copolyol.
Le mode opératoire est le suivant :
On dépose 0,75g de la composition sur une mèche de cheveux de 2,5 g. Après s'être lavé et rincé les mains, chaque testeur développe la mousse en malaxant la mèche. 9 testeurs sur 10 ont trouvé que la mousse de la composition de l'exemple 1 se développait plus vite, était plus abondante, moins compacte et avait une meilleur tenue que celle de la composition ne contenant pas le diméthicone copolyol.

Une composition contenant uniquement l'eau et le diméthicone copolyol ne présente aucun pouvoir moussant.

### EXEMPLE 2

On a préparé un shampooing conforme à l'invention qui présente la composition suivante :
- Lauryl éther sulfate de sodium à 2,2 moles d'oxyde d'éthylène en solution aqueuse à 28 % de MA 14 g MA
- Cocoyl amido propyl bétaïne en solution aqueuse à 25 % de MA (Tégobétaïne HS de GOLDSCHMIDT) 3 g MA
- Silicone oxyéthylénée de formule (II) dans laquelle : n=0, R₁=CH₃, R₂=(CH₂)₃O-(-C₂H₄O)₈-CH₃ (SILWET L77 de OSI) 0,5 g
- Alcool (C12-C15) polyoxyéthyléné à 2 moles d'oxyde de éthylène 3 g
- Polydiméthylsiloxane(PDMS)(Huile 47 V 500.000 de RHONE POULENC) 3 g
- NaCI 10 g
- Conservateurs, colorants, parfum qs
- HCI qs pH 5
- Eau qsp 100 g

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.

Ce shampooing présente de bonnes propriétés moussantes et apporte aux cheveux douceur et facilité de démêlage.

### EXEMPLE 3

On a préparé un shampooing conforme à l'invention qui présente la composition suivante :
- Lauryl éther sulfate de sodium à 2,2 moles d'oxyde d'éthylène en solution aqueuse à 28 % de MA 16,8 g MA
- Cocoyl bétaïne 2,7 g MA
- Silicone oxyéthylénée (FLUID DC 193 vendu par DOW CORNING) 0,5 g
- Alcool (C12-C15) polyoxyéthyléné à 2 moles d'oxyde de éthylène 3 g
- Polydiméthylsiloxane(PDMS)(Huile 47 V 500.000 vendu par RHONE POULENC) 3 g
- NaCI 10 g
- Conservateurs, colorants, parfum qs
- HCI qs pH 5
- Eau qsp 100 g

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.

Ce shampooing présente de bonnes propriétés moussantes et apporte aux cheveux douceur et facilité de démêlage.

### EXEMPLE 4

On a préparé un shampooing conforme à l'invention qui présente la composition suivante :
- Lauryl éther sulfate de sodium à 2,2 moles d'oxyde d'éthylène en solution aqueuse à 28 % de MA 16,8 g MA
- Cocoyl bétaïne en solution aqueuse à 30 % de MA 2,7 g MA
- Silicone oxyéthylénée de formule (II) (DC 193 vendu par DOW CORNING) 1 g
- Acétate de manganèse 12 g
- Conservateurs, colorants, parfum qs
- Eau qsp 100 g

Le pH spontané de la composition est de 5.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.

Le shampooing a été appliqué sur les cheveux mouillés, on a obtenu rapidement une mousse onctueuse et agréable.

### EXEMPLE 5

On a préparé un shampooing conforme à l'invention qui présente la composition suivante :
- Lauryl éther sulfate de sodium à 2,2 moles d'oxyde d'éthylène en solution aqueuse à 28 % de MA 16,8 g MA
- Cocoyl bétaïne en solution aqueuse à 30 % de MA 2,7 g MA
- Silicone oxyéthylénée de formule (II) (DC 193 vendu par DOW CORNING) 1 g
- Chlorure de lithium 12 g
- Conservateurs, colorants, parfum qs
- Eau qsp 100 g

Le pH spontané de la composition est de 5.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.

Le shampooing a été appliqué sur les cheveux mouillés, on a obtenu rapidement une mousse onctueuse et agréable.

### EXEMPLE 6

On a préparé un shampooing conforme à l'invention qui présente la composition suivante :
- Lauryl éther sulfate de sodium à 2,2 moles d'oxyde d'éthylène en solution aqueuse à 28 % de MA 16,8 g MA
- Cocoyl bétaïne en solution aqueuse à 30 % de MA 2,7 g MA
- Silicone oxyéthylénée de formule (II) ( DC 193 vendu par DOW CORNING) 1 g
- Chlorure de strontium 12 g
- Conservateurs, colorants, parfum qs
- Eau qsp 100 g

Le pH spontané de la composition est de 5.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.

Le shampooing a été appliqué sur les cheveux mouillés, on a obtenu rapidement une mousse onctueuse et agréable.

## Revendications

1. Composition cosmétique détergente caractérisée par le fait qu'elle comprend au moins un tensioactif anionique, au moins un cotensioactif non ionique ou amphotère, au moins une silicone oxyalkylénée et au moins un électrolyte présent à une concentration comprise entre 6 et 15% en poids par rapport au poids total de la composition et choisi parmi :
i) les nitrates de lithium, de strontium, de barium, d'yttrium, de néodyme, de gadolinium, de manganèse, de zinc ou de magnésium,
ii) les chlorures de lithium, de strontium, de barium, d'yttrium, de néodyme, de gadolinium, de manganèse, de zinc, de magnésium ou de sodium,
iii) les sulfates de calcium, de strontium ou de magnésium,
iv) les acétates de strontium, de manganèse ou de magnésium,
lesdits composés étant en quantité telle que la composition présente :
(a) un comportement rhéologique en écoulement caractérisé par un domaine de contraintes pour lesquelles la viscosité est constante, suivi d'un domaine de contraintes pour lequel la viscosité diminue lorsque la contrainte augmente, et
(b) une structure de phase lamellaire, cette phase étant apte à maintenir en suspension des particules non hydrosolubles éventuellement présentes dans la composition.

2. Composition selon la revendication 1, caractérisée en ce que les tensioactifs anioniques utilisables sont choisis parmi les sels des composés suivants : les alkylsulfates, les alkyléthersulfates, les alkylamidoéthersulfates, les monoglycérides sulfates, les alkylglycérylsulfonates, les alkylsulfonates, les alkylphosphates, les alkylamidesulfonates, les alkylarylsulfonates, les α-oléfinesulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acylaminoacides tels que les N-acylsarcosinates, les N-acylglutamates et les N-acyltaurates, les sels d'acides gras tels que les sels des acides undécylénique, oléique, ricinoléique, palmitique, stéarique, les acides d'huile de coprah ou d'huile de coprah hydrogénée, les acylhydroxyacides tels que les acyl-lactylates, les acides alkyl-D-galactoside uroniques et leurs sels, les acides éthers carboxyliques polyoxyalkylénés, en particulier ceux comportant de 2 à 24 groupements oxyde d'éthylène.

3. Composition selon l'une des revendications 1 ou 2, caractérisée en ce que les cotensioactifs non ioniques sont choisis parmi les alcools, les alphadiols, les alkylphénols ou les acides gras éthoxylés, propoxylés ou glycérolés, ces composés ayant une chaîne grasse comportant de 8 à 28 atomes de carbone, le nombre de groupements d'oxyde d'éthylène ou de propylène pouvant aller de 1 à 50 et celui de glycérol notamment de 1 à 30, les copolymères d'oxyde d'éthylène et de propylène, les condensats d'oxyde d'éthylène et de propylène sur des alcools gras, les amines ou les amides gras polyéthoxylés ayant de préférence de 2 à 30 moles d'oxyde d'éthylène, les amides gras polyglycérolés comportant en moyenne 1 à 5 groupements glycérol, les diglycolamides polyglycérolés, les esters d'acides gras du sorbitan éventuellement oxyéthylénés, les esters d'acides gras du sucrose, les esters d'acides gras polyoxyalkylénés, les alkylpolyglycosides éventuellement oxyalkylénés, les esters d'alkylglucosides, les dérivés de N-alkylglucamine et de N-acylméthylglucamines, les oxydes d'amine et leurs mélanges.

4. Composition selon l'une quelconque des revendications 1 ou 2, caractérisée en ce que les cotensioactifs amphotères sont choisis parmi les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant carboxylate, sulfonate, sulfate, phosphate ou phosphonate.

5. Composition selon la revendication 4, caractérisée en ce que les cotensioactifs amphotères sont choisis parmi les alkylbétaines, les alkyldiméthylbétaines, les alkyl sulfo bétaines, les alkylamidoalkyl bétaines, les alkylamidoalkyl sulfobétaines, les dérivés d'imidazoline tels que les dérivés d'amphocarboxyglycinate ou d'ampho-carboxypropionate et leurs mélanges.

6. Composition selon l'une des revendications précédentes, caractérisée par le fait que le ou les tensioactifs anioniques sont présents à raison de 3 à 50% en poids, de préférence de 5 à 30 % en poids par rapport au poids total de la composition.

7. Composition selon l'une quelconque des revendications précédentes, caractérisée par le fait que le ou les cotensioactifs sont présents à raison de 0,05 à 30% en poids, de préférence de 1 à 15 % en poids par rapport au poids total de la composition.

8. Composition selon la revendication précédente, caractérisée par le fait que l'électrolyte est présent à une concentration comprise entre 6 et 13 % en poids par rapport au poids total de la composition.

9. Composition selon l'une des revendications 1 à 8, caractérisée par le fait que l'électrolyte est le chlorure ou le nitrate de strontium, le chlorure de sodium

10. Composition selon l'une quelconque des revendications précédentes, caractérisée par le fait que les silicones modifiées par des groupements oxyalkylènes sont choisies parmi les composés de formules générales (I), (II), (III), (IV) et (V): 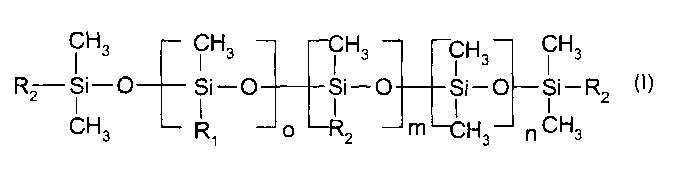 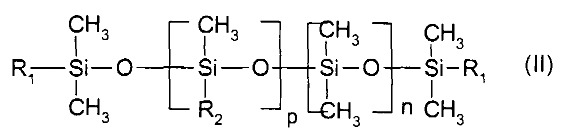 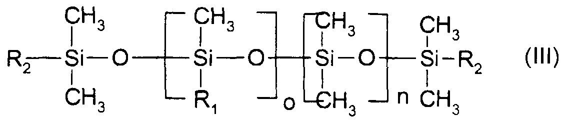 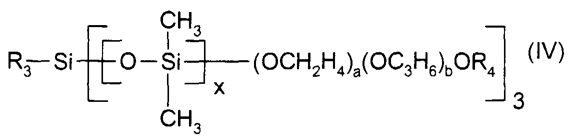 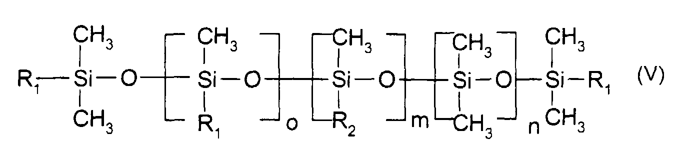 formules dans lesquelles :
- R₁, identique ou différent, représente un radical alkyle, linéaire ou ramifié, en C₁-C₃₀ ou phényle.
- R₂, identique ou différent, représente -C_{c}H_{2c}-O-(-C₂H₄O)ₐ(-C₃H₆O)_{b}-R₅ ou -C_{c}H_{2c}-O-(-C₄H₈O)ₐR₅,
- R₃, R₄, identiques ou différents, désignent un radical alkyle, linéaire ou ramifié, en C₁-C₁₂, et de préférence le radical méthyle,
- R₅, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 12 atomes de carbone, un radical hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, N(CH₂CH₂COOM)₂,aminoalkyle éventuellement substitué sur l'amine et sur la chaîne alkyle, carboxyacyle en C₁-C₃₀, un groupement phosphono éventuellement substitué par un ou deux radicaux aminoalkyle substitués, -CO(CH₂)_{d}COOM, -OCOCHR₇(CH₂)_{d}COOM, -NHCO(CH₂)_{d}OH, -NH₃Y,
- M, identique ou différent, désigne un atome d'hydrogène, Na, K, Li, NH₄ ou une amine organique,
- R₆ désigne un radical alkyle, linéaire ou ramifié, en C₁-C₃₀,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 1,
- c varie de 0 à 4,
- x varie de 1 à 100,
- Y représente un anion minéral ou organique monovalent.

11. Composition selon la revendication précédente, caractérisée par le fait que les silicones oxyalkylénées répondent à la formule générale (I) ou (II) et répondent à au moins une et de préférence toutes les conditions suivantes :
- c est égal 2 ou 3.
- R₁ désigne le radical méthyle.
- R₅ représente un atome d'hydrogène, un radical méthyle ou un radical acétyle et de préférence un atome d'hydrogène.
- a varie de 1 à 25 et plus particulièrement de 2 à 15.
- b est égal à 0.
- n varie de 0 à 100.
- p varie de 1 à 20.

12. Composition selon l'une quelconque des revendications précédentes, caractérisée en ce que la silicone oxyalkylénée est présente dans des concentrations comprises entre 0,01 et 10% en poids, de préférence entre 0,2 et 5% en poids par rapport au poids total de la composition.

13. Composition selon l'une quelconque des revendications précédentes, caractérisée par le fait que le rapport pondéral cotensioactif / tensioactif anionique est inférieur ou égal à 1.

14. Procédé de lavage de la peau ou des fibres kératiniques telles que les cheveux, caractérisé par le fait que l'on applique sur ceux-ci au moins une composition telle que définie dans l'une quelconque des revendications 1 à 13 puis que l'on effectue un rinçage.

## Patentansprüche

1. Reinigende kosmetische Zusammensetzung, dadurch gekennzeichnet, daß sie mindestens ein anionisches Tensid, mindestens ein nichtionisches oder amphoteres Cotensid, mindestens ein alkoxyliertes Silicon und mindestens einen Elektrolyten enthält, der in einer Konzentration von 6 bis 15 Gew.-%, bezogen auf das Gesamtgewicht der Zusammensetzung, enthalten ist und ausgewählt ist unter:
i) Lithiumnitrat, Strontiumnitrat, Bariumnitrat, Yttriumnitrat, Neodymnitrat, Gadoliniumnitrat, Mangannitrat, Zinknitrat und Magnesiumnitrat,
ii) Lithiumchlorid, Strontiumchlorid, Bariumchlorid, Yttriumchlorid, Neodymchlorid, Gadoliniumchlorid, Manganchlorid, Zinkchlorid, Magnesiumchlorid und Natriumchlorid,
iii) Calciumsulfat, Strontiumsulfat und Magnesiumsulfat,
iv) Strontiumacetat, Manganacetat und Magnesiumacetat,
wobei diese Verbindungen in einer solchen Menge enthalten sind, daß die Zusammensetzung
a) ein rheologisches Fließverhalten aufweist, das gekennzeichnet ist durch einen Spannungsbereich, in dem die Viskosität konstant ist, auf den ein Spannungsbereich folgt, in dem die Viskosität abnimmt, wenn die Spannung zunimmt, und
b) eine Struktur mit lamellarer Phase aufweist, wobei diese Phase imstande ist, nicht wasserlösliche Partikel, die gegebenenfalls in der Zusammensetzung enthalten sind, in Suspension zu halten.

2. Zusammensetzung nach Anspruch 1, dadurch gekennzeichnet, daß die verwendbaren anionischen grenzflächenaktiven Stoffe unter den Salzen der folgenden Verbindungen ausgewählt sind: Alkylsulfate, Alkylethersulfate, Alkylamidoethersulfate, Monoglyceridsulfate, Alkylglycerylsulfonate, Alkylsulfonate, Alkylphosphate, Alkylamidsulfonate, Alkylarylsulfonate, α-Olefinsulfonate, Alkylsulfosuccinate, Alkylethersulfosuccinate, Alkylamidsulfosuccinate, Alkylsulfosuccinamate, Alkylsulfoacetate, Alkyletherphosphate, Acylisethionate, N-Acylaminosäuren, wie die N-Acylsarcosinate, N-Acylglutamate und N-Acyltaurate, den Salzen von Fettsäuren, wie den Salzen der Undecylensäure, Ölsäure, Ricinoleinsäure, Palmitinsäure, Stearinsäure, die Säuren von Koprahöl oder von hydriertem Koprahöl, die Acylhydroxysäuren, wie die Acyllactylate, die Alkyl-D-Galactosid-Uronsäuren und ihre Salze, die polyethoxylierten Ethercarbonsäuren, und insbesondere die, die 2 bis 24 Ethylenoxid-Einheiten enthalten.

3. Zusammensetzung nach einem der Ansprüche 1 und 2, dadurch gekennzeichnet, daß die zusätzlichen nichtionischen grenzflächenaktiven Stoffe ausgewählt sind unter den ethoxylierten, propoxylierten, mit Glycerin veretherten Alkoholen, α-Diolen, Alkylphenolen oder Fettsäuren, wobei diese Verbindungen eine Fettkette enthalten, die 8 bis 28 Kohlenstoffatome aufweist, wobei die Zahl der Ethylenoxid- oder Propylenoxid-Einheiten im Bereich von 1 bis 50 liegen kann und die Zahl der Glycerin-Einheiten insbesondere 1 bis 30 betragen kann, den Ethylenoxid-Propylenoxid-Copolymeren, den Kondensationsprodukten von Ethylenoxid und Propylenoxid mit Fettalkoholen, den polyethoxylierten Fettaminen und Fettamiden, die vorzugsweise 2 bis 30 mol Ethylenoxid enthalten, den mehrfach mit Glycerin veretherten Fettamiden, die im Mittel 1 bis 5 Glycerin-Gruppen enthalten, den mehrfach mit Glycerin veretherten Diglykolamiden, den Estern aus Fettsäuren und Sorbitan, die gegebenenfalls ethoxyliert sind, den Estern aus Fettsäuren und Saccharose, den polyalkoxylierten Fettsäureestern, den gegebenenfalls alkoxylierten Alkylpolyglykosiden, den Alkylglucosidestern, den Derivaten von N-Alkylglucaminen und N-Acylmethylglucaminen, den Aminoxiden und ihren Gemischen.

4. Zusammensetzung nach einem der Ansprüche 1 und 2, dadurch gekennzeichnet, daß die amphoteren Cotenside unter den Derivaten von sekundären oder tertiären aliphatischen Aminen, deren aliphatischer Rest geradkettig oder verzweigt ist und 8 bis 22 Kohlenstoffatome aufweist und die mindestens eine wasserlöslich machende anionische Gruppe, wie eine Carboxy-, Sulfonat-, Sulfat-, Phosphat- oder Phosphonatgruppe enthalten, ausgewählt sind.

5. Zusammensetzung nach Anspruch 4, dadurch gekennzeichnet, daß die amphoteren Cotenside unter Alkylbetainen, Alkyldimethylbetainen, Alkylsulfobetainen, Alkylamidoalkylbetainen, Alkylamidoalkylsulfobetainen, den Imidazolinderivaten, wie den Amphocarboxyglycinat- und den Amphocarboxypropionat-Derivaten, und ihren Gemischen ausgewählt sind.

6. Zusammensetzung nach einem der vorhergehenden Ansprüche, dadurch gekennzeichnet, daß der oder die anionischen Tenside in einem Anteil von 3 bis 50 Gew.-%, vorzugsweise 5 bis 30 Gew.-%, bezogen auf das Gesamtgewicht der Zusammensetzung, enthalten sind.

7. Zusammensetzung nach einem der vorhergehenden Ansprüche, dadurch gekennzeichnet, daß das oder die Cotenside in einem Anteil von 0,05 bis 30 Gew.-%, vorzugsweise 1 bis 15 Gew.-%, bezogen auf das Gesamtgewicht der Zusammensetzung, enthalten sind.

8. Zusammensetzung nach dem vorhergehenden Anspruch, dadurch gekennzeichnet, daß der Elektrolyt in einer Konzentration von 6 bis 13 Gew.-%, bezogen auf das Gesamtgewicht der Zusammensetzung, enthalten ist.

9. Zusammensetzung nach einem der vorhergehenden Ansprüche 1 bis 8, dadurch gekennzeichnet, daß es sich bei dem Elektrolyten um Strontiumchlorid, Strontiumnitrat oder Natriumchlorid handelt.

10. Zusammensetzung nach einem der vorhergehenden Ansprüche, dadurch gekennzeichnet, daß die mit alkoxylierten Gruppen modifizierten Silicone unter den Verbindungen der allgemeinen Formeln (I), (II), (III), (IV) und (V) ausgewählt sind: 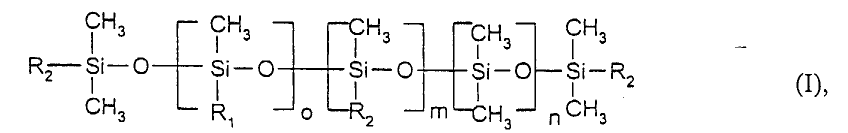 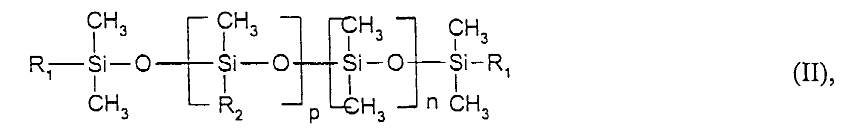 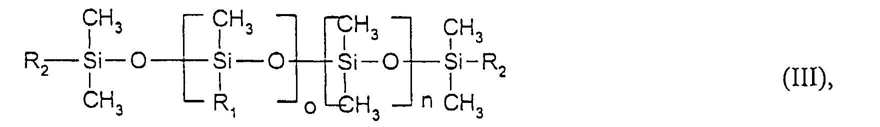 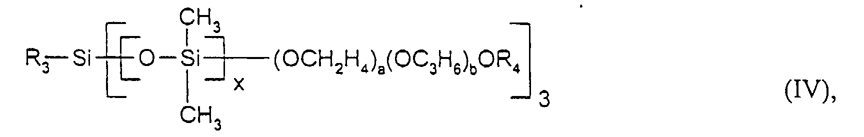 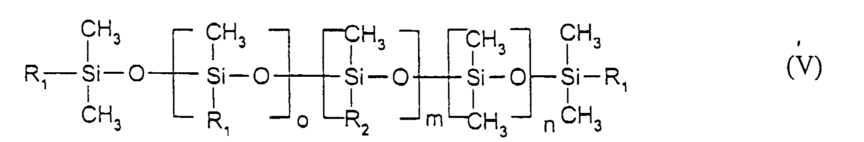 in denen bedeuten:
- die Reste R₁, die gleich oder verschieden sind, geradkettiges oder verzweigtes C₁ -C₃₀-Alkyl oder Phenyl,
- die Reste R₂, die gleich oder verschieden sind, -C₂H_{2c}-O-(-C₂H₄O)a(-C₃H₆O)_{b}-R₅ oder -C_{c}H_{2c}-O-(-C₄H₈O)ₐ-R₅
- die Reste R₃, R₄, die gleich oder verschieden sind, geradkettiges oder verzweigtes C₁-C₁₂-Alkyl und vorzugsweise Methyl,
- die Reste R₅, die gleich oder verschieden sind, Reste, die ausgewählt sind unter Wasserstoff, geradkettigem oder verzweigtem Alkyl mit 1 bis 12 Kohlenstoffatomen, geradkettigem oder verzweigtem Alkoxy mit 1 bis 6 Kohlenstoffatomen, geradkettigem oder verzweigtem Acyl mit 2 bis 12 Kohlenstofatomen, Hydroxy, -SO₃M, -OCOR₆, C₁-C₆-Aminoalkoxy, das gegenenfalls am Amin substituiert ist, C₂-C₆-Aminoacyl, das gegebenenfalls am Amin substituiert ist, -NHCH₂CH₂COOM, N(CH₂CH₂COOM)₂, Aminoalkyl, das gegebenenfalls am Amin und an der Alkylkette substituiert ist, C₁ -C₃₀-Carboxyacyl, Phosphono-Gruppen, die gegebenenfalls mit ein oder zwei substituierten Aminoalkylgruppen substituiert sind, -CO(CH₂)_{d}COOM, -OCOCHR₇(CH₂)_{d}COOM, -NHCO(CH₂)_{d}OH, -NH₃Y,
- die Reste M, die gleich oder verschieden sind, Wasserstoff, Na, K, Li, NH₄ oder ein organisches Amin,
- R₆ geradkettiges oder verzweigtes C₁-C₃₀-Alkyl,
- R₇ Wasserstoff oder die Gruppe -SO₃M,
- d eine Zahl im Bereich von 1 bis 10,
- m eine Zahl im Bereich von 0 bis 20,
- n eine Zahl im Bereich von 0 bis 500,
- o eine Zahl im Bereich von 0 bis 20,
- p eine Zahl im Bereich von 1 bis 50,
- a eine Zahl im Bereich von 0 bis 50,
- b eine Zahl im Bereich von 0 bis 50,
- a + b zusammen mindestens 1,
- c eine Zahl im Bereich von 0 bis 4,
- x eine Zahl im Bereich von 1 bis 100,
- Y ein einwertiges anorganisches oder organisches Anion.

11. Zusammensetzung nach dem vorhergehenden Anspruch, dadurch gekennzeichnet, daß die alkoxylierten Silicone der allgemeinen Formel (I) oder (II) entsprechen und mindestens eine der folgenden Bedingungen und vorzugsweise alle folgenden Bedingungen erfüllen:
- c ist die Zahl 2 oder 3,
- R₁ bedeutet Methyl,
- R₅ stellt Wasserstoff, Methyl, Acetyl und vorzugsweise Wasserstoff dar,
- a liegt im Bereich von 1 bis 25 und insbesondere 2 bis 15,
- b ist gleich Null,
- n liegt im Bereich von 0 bis 100,
- p liegt im Bereich von 1 bis 20.

12. Zusammensetzung nach einem der vorhergehenden Ansprüche, dadurch gekennzeichnet, daß das alkoxylierte Silicon in einer Konzentration von 0,01 bis 10 Gew.-%, vorzugsweise 0,2 bis 5 Gew.-%, bezogen auf das Gesamtgewicht der Zusammensetzung, enthalten ist.

13. Zusammensetzung nach einem der vorhergehenden Ansprüche, dadurch gekennzeichnet, daß das Gewichtsverhältnis des Cotensids zum anionischen Tensid höchstens 1 beträgt.

14. Verfahren zum Waschen der Haut oder von Keratinfasern, wie der Haare, dadurch gekennzeichnet, daß auf die Haut oder die Keratinfasern mindestens eine Zusammensetzung, die wie in einem der Ansprüche 1 bis 13 definiert ist, aufgetragen wird und anschließend diese Zusammensetzung von der Haut oder den Keratinfasern abgespült wird.

## Claims

1. Detergent cosmetic composition, characterized in that it comprises at least one anionic surfactant, at least one non-ionic or amphoteric cosurfactant, at least one oxyalkylenated silicone and at least one electrolyte present at a concentration of between 6 and 15% by weight with respect to the total weight of the composition and chosen from:
i) lithium, strontium, barium, yttrium, neodymium, gadolinium, manganese, zinc or magnesium nitrates,
ii) lithium, strontium, barium, yttrium, neodymium, gadolinium, manganese, zinc, magnesium or sodium chlorides,
iii) calcium, strontium or magnesium sulphates,
iv) strontium, manganese or magnesium acetates, the said compounds being in amounts such that the composition exhibits :
(a) flow rheological behaviour characterized by a range of stresses for which the viscosity is constant, followed by a range of stresses for which the viscosity decreases as the stress increases, and
(b) a lamellar phase structure, this phase being capable of keeping in suspension water-insoluble particles optionally present in the composition.

2. Composition according to Claim 1, characterized in that the anionic surfactants which can be used are chosen from the salts of the following compounds: alkyl sulphates, alkyl ether sulphates, alkylamido ether sulphates, monoglyceride sulphates, alkylglyceryl sulphonates, alkyl sulphonates, alkyl phosphates, alkylamide sulphonates, alkylaryl sulphonates, α-olefin sulphonates, alkyl sulphosuccinates, alkyl ether sulphosuccinates, alkylamide sulphosuccinates, alkyl sulphosuccinamates, alkyl sulphoacetates, alkyl ether phosphates, acylisethionates or N-acylamino acids, such as N-acylsarcosinates, N-acylglutamates and N-acyl-taurates, salts of fatty acids, such as the salts of undecylenic, oleic, ricinoleic, palmitic or stearic acids, coconut oil acid or hydrogenated coconut oil acid, or acyl hydroxy acids, such as acyllactylates, alkyl D-galactosiduronic acids and their salts, or polyoxyalkylenated ether carboxylic acids, in particular those containing from 2 to 24 ethylene oxide groups.

3. Composition according to one of Claims 1 and 2, characterized in that the non-ionic cosurfactants are chosen from alcohols, α-diols, alkyl phenols or fatty acids which are ethoxylated, propoxylated or glycerolated, these compounds having a fatty chain containing from 8 to 28 carbon atoms, it being possible for the number of ethylene or propylene oxide groups to range from 1 to 50 and that of glycerol groups to range in particular from 1 to 30, copolymers of ethylene oxide and of propylene oxide, condensates of ethylene oxide and of propylene oxide with fatty alcohols, polyethoxylated fatty amides or amines preferably having from 2 to 30 mol of ethylene oxide, polyglycerolated fatty amides containing on average 1 to 5 glycerol groups, polyglycerolated diglycolamides, optionally oxyethylenated fatty acid esters of sorbitan, fatty acid esters of sucrose, polyoxyalkylenated fatty acid esters, optionally oxyalkylenated alkylpolyglycosides, alkylglucoside esters, N-alkylglucamine and N-acyl-methylglucamine derivatives, amine oxides and their mixtures.

4. Composition according to either of Claims 1 and 2, characterized in that the amphoteric cosurfactants are chosen from secondary or tertiary aliphatic amine derivatives in which the aliphatic radical is a linear or branched chain containing 8 to 22 carbon atoms and containing at least one carboxylate, sulphonate, sulphate, phosphate or phosphonate water-solubilizing anionic group.

5. Composition according to Claim 4, characterized in that the amphoteric cosurfactants are chosen from alkyl betaines, alkyldimethyl betaines, alkyl sulphobetaines, alkylamidoalkyl betaines, alkylamidoalkyl sulphobetaines, imidazoline derivatives, such as amphocarboxyglycinate or amphocarboxypropionate derivatives, and their mixtures.

6. Composition according to one of the preceding claims, characterized in that the anionic surfactant or surfactants are present in the proportion of 3 to 50% by weight, preferably of 5 to 30% by weight, with respect to the total weight of the composition.

7. Composition according to any one of the preceding claims, characterized in that the cosurfactant or cosurfactants are present in the proportion of 0.05 to 30% by weight, preferably of 1 to 15% by weight, with respect to the total weight of the composition.

8. Composition according to the preceding claim, characterized in that the electrolyte is present at a concentration of between 6 and 13% by weight with respect to the total weight of the composition.

9. Composition according to one of Claims 1 to 8, characterized in that the electrolyte is Strontium chloride or nitrate or sodium chloride.

10. Composition according to any one of the preceding claims, characterized in that the silicones which are modified by oxyalkylene groups are chosen from compounds of general formulae (I), (II) , (III), (IV) and (V) : 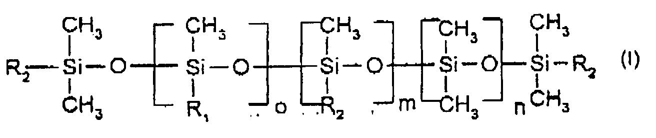 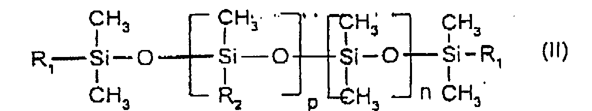 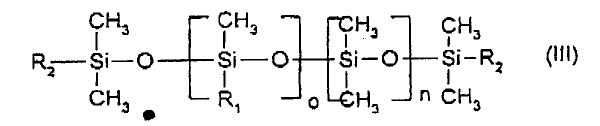 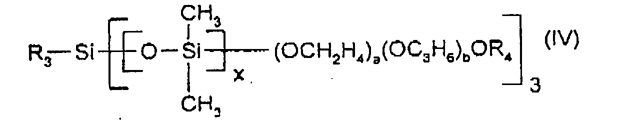 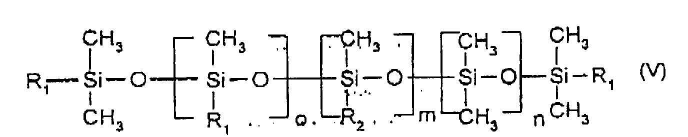 in which formulae:
- R₁, which is identical or different, represents a linear or branched C₁-C₃₀ alkyl radical or a phenyl radical,
- R₂, which is identical or different, represents -C_{c}H_{2c}-O-(-C₂H₄O)ₐ(-C₃H₆O)_{b}-R₅ or -C_{c}H_{2c}-O-(-C₄H₈O)ₐR₅,
- R₃ and R₄, which are identical or different, denote a linear or branched C₁-C₁₂ alkyl radical and preferably the methyl radical,
- R₅, which is identical or different, is chosen from a hydrogen atom, a linear or branched alkyl radical containing 1 to 12 carbon atoms, a linear or branched alkoxy radical containing 1 to 6 carbon atoms, a linear or branched acyl radical containing 2 to 12 carbon atoms, a hydroxyl radical, -SO₃M, -OCOR₆, C₁-C₆ aminoalkoxy optionally substituted on the amine, C₂-C₆ aminoacyl optionally substituted on the amine, -NHCH₂CH₂COOM, N(CH₂CH₂COOM)₂, aminoalkyl optionally substituted on the amine and on the alkyl chain, C₁-C₃₀ carboxyacyl, a phosphono group optionally substituted by one or two substituted aminoalkyl radicals, -CO(CH₂)_{d}COOM, -OCOCHR₇(CH₂)_{d}COOM, -NHCO(CH₂)_{d}OH or -NH₃Y,
- M_{,} which is identical or different, denotes a hydrogen atom, Na, K, Li, NH₄ or an organic amine,
- R₆ denotes a linear or branched C₁-C₃₀ alkyl radical,
- R₇ denotes a hydrogen atom or an SO₃M radical,
- d varies from 1 to 10,
- m varies from 0 to 20,
- n varies from 0 to 500,
- o varies from 0 to 20,
- p varies from 1 to 50,
- a varies from 0 to 50,
- b varies from 0 to 50,
- a + b is greater than or equal to 1,
- c varies from 0 to 4,
- x varies from 1 to 100,
- Y represents a monovalent inorganic or organic anion.

11. Composition according to the preceding claim, characterized in that the oxyalkylenated silicones correspond to the general formula (I) or (II) and correspond to at least one, and preferably all, of the following conditions:
- c is equal to 2 or 3,
- R₁ denotes the methyl radical,
- R₅ represents a hydrogen atom, a methyl radical or an acetyl radical and preferably a hydrogen atom,
- a varies from 1 to 25 and more particularly from 2 to 15,
- b is equal to 0,
- n varies from 0 to 100,
- p varies from 1 to 20.

12. Composition according to any one of the preceding claims, characterized in that the oxyalkylenated silicone is present in concentrations of between 0.01 and 10% by weight, preferably between 0.2 and 5% by weight, with respect to the total weight of the composition.

13. Composition according to any one of the preceding claims, characterized in that the cosurfactant/anionic surfactant ratio by weight is less than or equal to 1.

14. Process for washing the skin or keratinous fibres, such as the hair, characterized in that at least one composition as defined in any one of Claims 1 to 13 is applied on them and in that rinsing is then carried out.
